(19) Europäisches Patentamt
European Patent Office
Office européen des brevets

(11) Veröffentlichungsnummer: **0 086 375**
**B1**

(12) # EUROPÄISCHE PATENTSCHRIFT

(45) Veröffentlichungstag der Patentschrift: **09.04.86**

(51) Int. Cl.⁴: **C 07 D 213/64**, C 07 D 213/79, A 01 N 43/40, C 07 D 213/68

(21) Anmeldenummer: **83100757.0**

(22) Anmeldetag: **27.01.83**

(54) **Pyridinderivate, ihre Herstellung und Verwendung.**

(30) Priorität: **13.02.82 DE 3205150**

(43) Veröffentlichungstag der Anmeldung: **24.08.83 Patentblatt 83/34**

(45) Bekanntmachung des Hinweises auf die Patenterteilung: **09.04.86 Patentblatt 86/15**

(84) Benannte Vertragsstaaten: **AT BE CH DE FR GB IT LI NL**

(56) Entgegenhaltungen:
**DE - A - 1 542 736**
**US - A - 3 576 616**

**Die Akte enthält technische Angaben, die nach dem Eingang der Anmeldung eingereicht wurden und die nicht in dieser Patentschrift enthalten sind.**

(73) Patentinhaber: **CELAMERCK GmbH & Co. KG, Binger Strasse 173, D-6507 Ingelheim am Rhein (DE)**

(72) Erfinder: **Mengel, Rudolf, Dr. Dipl.-Chem., Schützenpfad 22, D-6507 Ingelheim (DE)**
Erfinder: **Schröder, Ludwig, Dr. Dipl.-Chem., Frankenstrasse 7, D-6507 Ingelheim (DE)**
Erfinder: **Stransky, Werner, Dr. Dipl.-Chem., Im Hippel 24, D-6535 Gau-Algesheim (DE)**
Erfinder: **Linden, Gerbert, Dr. Dipl.-Landwirt, Turnierstrasse 44, D-6507 Ingelheim (DE)**
Erfinder: **Lust, Sigmund, Dr., Klappacher Strasse 2f, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 086 375 B1**

LIBER, STOCKHOLM 1986

**Beschreibung**

Die Erfindung betrifft neue Pyridinderivate der Formel

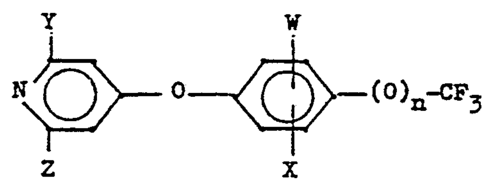 (I),

in der

W und R, die gleich oder verschieden sein können, für H, F, Cl, Br, J, $NO_2$ oder einen gegebenenfalls fluor- oder chlorsubstituierten $C_1$-$C_4$-Alkylrest,

Y für H, F, Cl, Br oder einen gegebenenfalls fluor- oder chlorsubstituierten $C_1$-$C_4$-Alkylrest,

Z für F, Cl, Br, einen gegebenenfalls fluor- oder chlorsubstithierten $C_1$-$C_4$-Alkylrest oder COOH oder COO- ($C_1$-$C_4$-Alkyl)

und

n für 0 oder 1 steht,

die Herstellung der neuen Verbindungen und ihre Verwendung als biozide, insbesondere herbizide Wirkstoffe.

Soweit die aliphatischen Reste 3 oder 4 C-Atome enthalten, können sie unverzweigt oder verzweigt sein. Als fluor- oder chlorsubstituierte Alkylreste sind vor allem $CF_3$ und $CCl_3$ zu nennen.

Als bevorzugte Substituentenbedeutungen sind zu nennen:

W gleich H, Cl, F, $CH_3$, $CF_3$, $NO_2$;

X gleich H, F, Cl, $CH_3$, $CF_3$, $NO_2$;

Y gleich H, F, Cl, $CF_3$, $CCl_3$, $CH_3$;

Z gleich F, Cl, $CF_3$, $CH_3$, COOH, $COOCH_3$, $COOC_2H_5$

n steht bevorzugt für 0.

Im Falle Z = COOH können die neuen Säuren auch als Salze, insbesondere als Alkalisalze vorliegen.

Aus der DE-OS 15 42 736 sind aryloxysubstituierte Pyridine bekannt, die der allgemeinen Formel

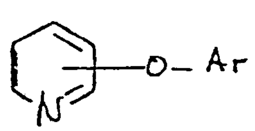

entsprechen. Diese Verbindungen, die im Pyridinring keinen zusätzlichen Substituenten aufweisen - beispielsweise die besonders herausgestellte Verbindung 4-(2',4'-Dichlorphenoxy)-pyridin-sind herbizid wirksam.

Außerdem sind aus der US-PS 35 76 616 herbizid wirksame phenoxysubstituierte Pyridine der allgemeinen Formel

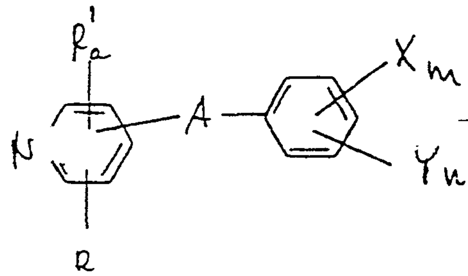

bekannt die im Pyridinteil mindestens einfach mit Fluor, Chlor oder Brom und im Phenoxyteil ein- oder mehrfach mit Chlor oder Brom substituiert sind.

Die erfindungsgemäßen Verbindungen zeichnen sich demgegenüber aus durch eine starke Wirkung gegen zahlreiche monokotyle und dikotyle Unkräuter z. B. auch schwer bekämpfbare wie Galium aparine, Verinica hederifolia. Von erheblichem Nutzen ist auch die Möglichkeit, für die Praxis bedeutsame Wirkungslücken vieler sonst sehr bewährter Handelsprodukte zu schließen, indem man die neuen Wirkstoffe gemeinsam mit ihnen anwendet.

Kombinationspartner dieser Art sind beispielsweise: Chlortoluron und verwandte Harnstoffderivate, Terbutryn und verwandte Triazinderivate, Trifluralin und verwandte Derivate, Alachlor, Dimethachlor und verwandte Verbindungen.

Die neuen Verbindungen werden in an sich bekannter Weise hergestellt.

Man setzt dazu ein Pyridinderivat der Formel

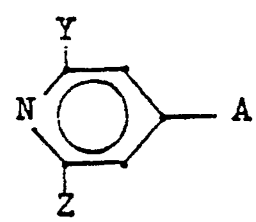 (II)

(A gleich Halogen, insbesondere Chlor, Fluor oder $NO_2$)

mit einem Phenol oder Phenolat der Formel

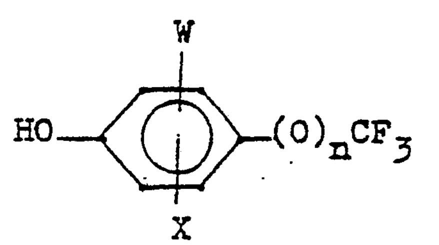 bzw.

(IIIa)

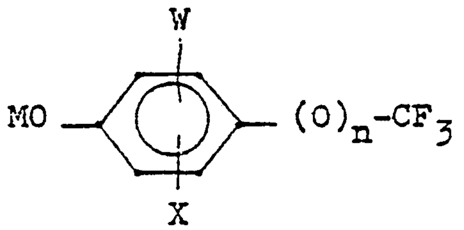

(IIIb)

(M gleich 1 Äquivalent eines Kations, vorzugsweise $Na^+$, $K^+$, 1/2 $Ca^{2+}$) um.

Die Umsetzung der Verbindungen II und IIIa bzw. III b erfolgt bei Temperaturen zwischen der Umgebungstemperatur und etwa 160°C. Man arbeitet vorzugsweise in polaren Lösungsmitteln wie Aceton, Butan-2-on, Acetonitril, Dimethylformamid, Dimethylsulfoxid bei Raumtemperatur oder mäßig erhöhter Temperatur. Bei der Umsetzung der Phenole gibt man säurebindende Mittel, z.B. Alkalicarbonate, etwa $Na_2CO_3$, $K_2CO_3$, oder Alkali- oder Erdalkalihydroxide (NaOH, KOH, $Ca(OH)_2$ zu. Um eine günstige Ausbeute zu erzielen, kann es vorteilnaft sein, das Phenol bzw. dessen Salze und/oder das säurebindende Mittel im Überschuß einzusetzen.

Die Ausgangsstoffe der Formeln II, IIIa und IIIb sind bekannt oder können analog den bekannten Verbindungen erhalten werden.

Die Verbindunen der Formel I werden für die Anwendung im Pflanzenschutz mit gebräuchlichen Hilfs- und/ oder Trägerstoffen zu üblichen Formulierungen verarbeitet.

Die Aufwandmengen bei der Anwendung als Herbizide liegen für die neuen Verbindungen im allgemeinen zwischen etwa 0, 05 und 2 kg, vorzugsweise 0, 1 und 1 kg, pro Hektar, je nach der verwendeten Substanz und dem zu bekämpfenden Unkraut. In Kombination mit anderen Herbiziden genügen im allgemeinen noch geringere Mengen (bis hinunter zu etwa 0, 03 kg/ha) der er-findungsgemäßen Verbindungen. Die nachstehenden Zubereitungen stellen beispiele für die Formulierung der neuen herbiziden Mittel dar (Angaben in Gewichtsprozent):

**a) Suspensionspulver 1**

25 % Wirkstoff gemäß Formel I
55 % Kaolin
9 % Ligninsulfonat als Dispergiermittel
1 % Natriumtetrapropylenbenzolsulfonat als Netzmittel

**b) Suspensionpulver 2**

80 % Wirkstoff gemäß Formel I
8 % Calciumligninsulfonat
5 % kolloidale Kieselsäure
5 % Natriumsulfat
2 % Natriumdiisobutylnaphtinsulfonat

**c) Emulsionskonzentrat**

40 % Wirkstoff gemäß Formel I
25 % Sellsol A (flüssiges Gemisch aromatischer Kohlenwasserstoffe)
25 % N-Methylpyrrolidon
10 % Emulsogen I 40 (anionenaktiver Emulgator)

Die unter a) bis c) angegebenen Konzentrate werden für die Anwendung mit Wasser auf etwa 0,05 bis 5 Gewichtsprozent verdünnt.

Die Wirkung der neuen Verbindungenwurde im Gewächshausversuch mit der Aufwandmenge 2 kg/ha geprüft (Vorauflaufwerte).

Als Vergleich diente A (= 4-(2, 4-Dichlorphenoxy)pyridin nach der DE-OS 15 43 736.

Die Bewertungerfolgte nach den neunstufigen Bemitierungsschlüssel, wobei 1 = 100 % Wirkung, 9 = keine Wirkung bedeutet.

| Verbindung | SIN* | LYC* | ECH* |
|---|---|---|---|
| A (Stand der Technik) | 9 | 9 | 9 |
| erfindungsgemäß | | | |
| Beispiel 1 | 1 | 1 | 1 |
| Beispiel 2 | 1 | 1 | 1 |

* SIN = Sinapis alba
LYC = Lycopersicum esculentum
ECH = Echinocloa crus galli

Mit der guten Wirksamkeit der neuen Verbindungen verbindet sich eine gute Verträglichkeit in zahlreichen Kulturen (z.B. Hafer, Weizen, Gerste, Kartoffeln, Soja, Baumwolle, Erbse), wodurch ein selektiver Einsatz ermöglicht wird.

Die Anwendung kann sowohl vor wie auch nach dem Auflaufen erfolgen. Werden die neuen Wirkstoffe allein angewendet, so geschleht das vorzugsweise vor dem Auflaufen.

Für Kombinationen, vor allen für die Nachauflaufbehandlung, eignen sich besonders

1. Wuchsstoffherbizide, etwa 2, 4-DP, MCPA, CMPP;

2. Kontaktherbizide, etwa Ioxynil, Bromoxynil,

Bentazon, Bromphenoxim, Dinoterb;
3. Grasherbizide, etwa Isoproturon, Diclofopmethyl. Die folgenden Beispiele sollen die erfindungsgemäßen Herstellverfanren näher erläutern, ohne sie zu beschränken:

**Beispiel 1**

2, 6-Dichlor-4- (2-chlor-4-tri fluormethylphenoxy)pyridin

Zu einer Suspension von 19,65 g (0,1 Mol) 2-Chlor-4-trifluormethyl-phenol und 19,29 g (0,1 Mol) 2,6

Dichlor-4-mitropyridin in 30 ml Dimethylformamid werden unter Eiskühlung und Rühren portionsweise 16,5 g (0,12 Mol) $K_2CO_3$ hinzugefügt. Nach 2-stüdigem Rühren bei Raumtemperatur wird die Mischung in 250 ml Eiswasser eingerüht. Zunächst scheidet sich ein Öl ab, das nach einiger Zeit kristallisiert. Das Produkt wird abgesaugt, mit Wasser gewaschen und anschließend in Chloroform gelöst. Man schüttelt mit Wasser, trennt die organische Phase ab und engt diese nach dem Trocknen mit $Na_2SO_4$ ein. Man erhält 25,3 g (75%) farblose Sbstanz vom Schmelzpunkt 66° C.

**Beispiel 2**

2-Chlor-6-methyl-4-(2-chlor-4-trifluormethylphenoxy)pyridin

Eine Lösung von 19,65 g (0,1 Mol) 2-Chlor-4-trifluormethyl-phenol sowie 17, 25 g (0,1 Mol) 2-Chlor-6-methyl-4-nitro-pyridin in 40 ml Dimethylformamid wird unter Rühren portionsweise mit 16,5 g (0,12 Mol) $K_2CO_3$ versetzt, auf 80° C erwärmt und 3 Stunden bei dieser Temperatur gehalten. Nach dem Abkühlen gießt man auf 200 ml Eiswasser und extrahiert mit $CHCl_3$. Die organische Phase wird abgetrennt, mit Wasser gewaschen, mit $Na_2SO_4$ getrocknet und eingeengt. Der Rückstand wird durch Säulenchromatographie (Kieselgel) gereinigt. Man erhält ein zähes Öl, das nach einiger Zeit kristallisiert. Ausbeute: 20,9 g (65% d.Th.), Fp. 75° C.

Analyse: $C_{13}H_8Cl_2F_3NO$ (322.11)
ber: C 48,5% H 2,5% N 4,35 %
gef: C 47,92% H 2,27 % N 4,75 %

**Beispiel 3**

2-Chlor-4- (2-chlor-4-trifluormethylphenoxy) - pyridin

Eine Lösung von 2,95 g (0, 015 Mol) 2-Chlor-4-trifluormethylphenol sowie 2, 2 g (0, 015 Mol) 2-Chlor-4-nitro-pyridin in 10 ml Dimethylformamid wird unter Rühren portionsweise mit 2,48 g (0,018 Mol) $K_2CO_3$ versetzt, 1 Stunde bei Raumtemperatur und 1,5 Stunden bei 80° C gerührt. Anschließend wird auf Eiswasser gegossen und mit Chloroform extranert. Die organische Phase wird abgetrennt, mit 2 n NaOH und Wasser gewaschen, mit $Na_2SO_4$ getrocknet und eingeengt.

Man erhält ein farbloses Öl.

Analyse: $C_{16}H_6Cl_2F_3NO$
ber: C 46,7 % H 1,96 % Cl 23,01 %
gef: C 46,03 % H 1,61 % Cl 22,67 %

1H-NMR ($CDCl_3$, ppm)

Protonen im Pyridinteil: 1 Proton bei 8,22 ppm, 2 Protonen bei 6,71. Protonen im Phenylreil: je 1 Proton bei 7,76, 7,56 und 7,22 ppm.

**Beispiel 4**

2-Chlor-6-methoxycarbonyl-4-(2-chlor-4-trifluormethylphenoxy) - pyridin

2,06 g (0,01 mol) 4,6-Dichlorpicolinsäuremethylester, 2,15 g (0,011 mol) 2-Chlor-4-trifluormethylphenol, 1,5 g pulverisiertes Kaliumcarbonat und 5 ml Dimethylformamid werden unter Rühren eine Stunde auf 120° C erwärmt. Das Gemisch erstarrt. Es wird mehrmals mit Eiswasser verrührt und dekantiert, dann mit verdünnter Essigsäure angesäuert und mit Essigester extrahiert. Die organische Phase wird abgetrennt, mit Wasser gewaschen und mit Natriumsulfat getrocknet. Man destilliert das Lösungsmittel ab und reinigt das resultierende Öl chromatographisch über 40 g Kieselgel (Laufmittel Diisopropylether). Das gereinigte Produkt ist ein helles Öl; Ausbeute 1,4 g (38 % d.Th.). $R_f$-Wert 0,48 (DC-Fertigplatten der Firma E. MERCK, Darmstadt, mit Kieselgel 60-F-254. Laufmittel Aceton/Diisopropylether 1: 2; die Flecken sind in ultravioletten Licht bei 254 µm sichtbar). Die DC-Fertigplatten des hier verwendeten Typs und das uv-Licht der genannten Wellenlänge dienten auch zur Bestimmung der übrigen $R_f$-Werte.

Die folgenden Verbindungen können beispielsweise gemäß der Erfindung erhalten werden:

**Tabelle I**

Formel:

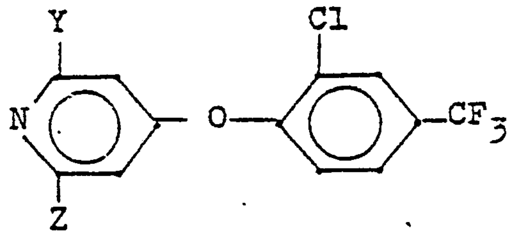

| Nr. | Y | Z | physikal. Kenndaten |
|---|---|---|---|
| 1 | H | $CH_3$ | |
| 2 | $CH_3$ | $CH_3$ | |
| 3 | H | $CF_3$ | |
| 4 | H | F | |
| 5 | F | F | Kp. 112-114°C/0,05 mbar |
| 6 | Cl | $CF_3$ | |
| 7 | F | $CH_3$ | |
| 8 | Cl | $CCl_3$ | |
| 9 | F | $CCl_3$ | |
| 10 | H | Br | |
| 11 | Br | Br | |
| 12 | $CH_3$ | Br | |
| 13 | $CCl_3$ | Br | |
| 14 | Br | $CF_3$ | |
| 15 | H | $OCH_3$ | |
| 16 | Cl | $OCH_3$ | |
| 17 | H | $O-n-C_4H_9$ | |
| 18 | H | COOH | |
| 19 | H | $COOC_2H_5$ | |
| 20 | Cl | COONa | |

**Tabelle II**

Formel

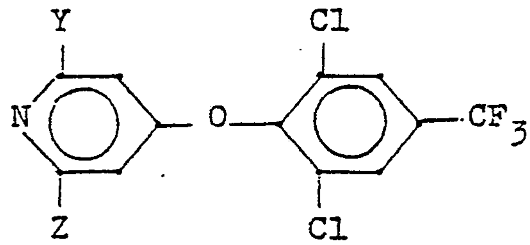

| Nr. | Y | Z | physikal. Kenndaten |
|---|---|---|---|
| 1 | H | $CH_3$ | |
| 2 | $CH_3$ | $CH_3$ | |
| 3 | H | $CF_3$ | |
| 4 | H | F | |
| 5 | F | F | |
| 6 | Cl | $CF_3$ | |
| 7 | F | $CH_3$ | |
| 8 | Cl | $CCl_3$ | |
| 9 | F | $CCl_3$ | |
| 10 | H | Br | |
| 11 | Br | Br | |
| 12 | $CH_3$ | Br | |
| 13 | $CCl_3$ | Br | |
| 14 | Br | $CF_3$ | |
| 15 | H | $OCH_3$ | |
| 16 | Cl | $OCH_3$ | |
| 17 | H | $O-n-C_4H_9$ | |
| 18 | H | $CH(CH_3)_2$ | |
| 19 | H | CCOK | |
| 20 | Cl | Cl | Öl $R_f$-Wert 0,58 1) |
| 21 | Cl | $COOCH_3$ | |

1) Laufmittel Diisopropylether/Benzin 1 : 1

**Tabelle III**

Formel

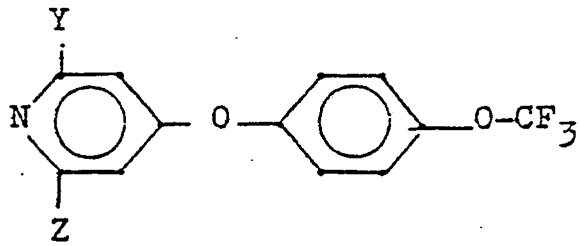

| Nr. | Y | Z | physikal.Kenndaten | |
|---|---|---|---|---|
| 1 | H | $CH_3$ | | |
| 2 | $CH_3$ | $CH_3$ | | |
| 3 | H | $CF_3$ | | |
| 4 | H | F | | |
| 5 | F | F | | |
| 6 | Cl | $CF_3$ | | |
| 7 | F | $CH_3$ | | |
| 8 | Cl | Cl | Öl | $R_f$-Wert 0,64 1) |
| 9 | F | $CCl_3$ | | |
| 10 | H | Br | | |
| 11 | Br | Br | | |
| 12 | $CH_3$ | Br | | |
| 13 | $CCl_3$ | Br | | |
| 14 | Br | $CF_3$ | | |
| 15 | H | $OCH_3$ | | |
| 16 | Cl | $OCH_3$ | | |
| 17 | H | $O-n-C_4H_9$ | | |
| 18 | $C_2H_5$ | $C_2H_5$ | | |
| 19 | H | $C(CH_3)_3$ | | |
| 20 | H | COOH | | |
| 21 | Cl | $CH_3$ | Öl | $R_f$-Wert 0,52 2) |
| 22 | H | Cl | Öl | $R_f$-Wert 0,54 1) |

1) Laufmittel Diisopropylether/Aceton 5 : 1
2) Laufmittel Diisopropylether

**Tabelle IV**

Formel

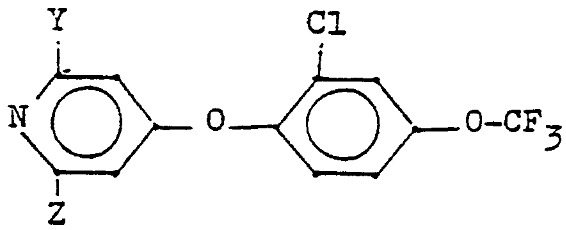

| Nr. | Y | Z | physikal. Kenndaten |
|---|---|---|---|
| 1 | H | $CH_3$ | |
| 2 | $CH_3$ | $CH_3$ | |
| 3 | H | $CF_3$ | |
| 4 | H | F | |
| 5 | F | F | |
| 6 | Cl | $CF_3$ | |
| 7 | F | $CH_3$ | |
| 8 | Cl | $CCl_3$ | |
| 9 | F | $CCl_3$ | |
| 10 | H | Br | |
| 11 | Br | Br | |
| 12 | $CH_3$ | Cl | Fp. 90°C |
| 13 | $CCl_3$ | Br | |
| 14 | Br | $CF_3$ | |
| 15 | H | $OCH_3$ | |
| 16 | Cl | $OCH_3$ | |
| 17 | H | $O-n-C_4H_9$ | |
| 18 | H | $CH(CH_3)_2$ | |
| 19 | H | COOH | |
| 20 | H | $CCOC_2H_5$ | |
| 21 | Cl | Cl | Fp. 92°C |

Tabelle V

Formel

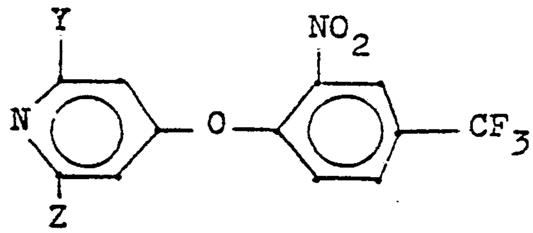

| Nr. | Y | Z | physikal. Kenndaten |
|---|---|---|---|
| 1 | H | $CH_3$ | |
| 2 | $CH_3$ | $CH_3$ | |
| 3 | H | $CF_3$ | |
| 4 | H | F | |
| 5 | F | F | |
| 6 | Cl | $CF_3$ | |
| 7 | F | $CH_3$ | |
| 8 | Cl | $CCl_3$ | |
| 9 | F | $CCl_3$ | |
| 10 | H | Br | |
| 11 | Br | Br | |
| 12 | $CH_3$ | Br | |
| 13 | $CCl_3$ | Br | |
| 14 | Br | $CF_3$ | |
| 15 | H | $OCH_3$ | |
| 16 | Cl | $OCH_3$ | |
| 17 | H | $O-n-C_4H_9$ | |
| 18 | Cl | Cl | Fp. 113 - 115°C |
| 19 | $CH_3$ | Cl | |
| 20 | H | COOH | |
| 21 | Cl | $COOCH_3$ | |

Tabelle VI

Formel

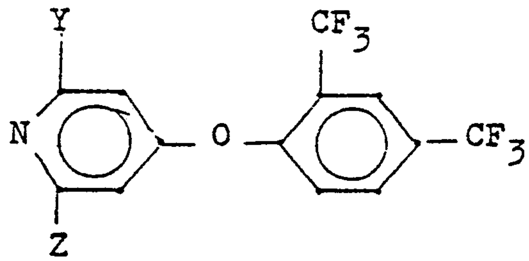

| Nr. | Y | Z |
|---|---|---|
| 1 | H | $CH_3$ |
| 2 | $CH_3$ | $CH_3$ |
| 3 | H | $CF_3$ |
| 4 | H | F |
| 5 | F | F |
| 6 | Cl | $CF_3$ |
| 7 | F | $CH_3$ |
| 8 | Cl | $CCl_3$ |
| 9 | F | $CCl_3$ |
| 10 | H | Br |
| 11 | Br | Br |
| 12 | $CH_3$ | Br |
| 13 | $CCl_3$ | Br |
| 14 | Br | $CF_3$ |
| 15 | H | $OCH_3$ |
| 16 | Cl | $OCH_3$ |
| 17 | H | $O-n-C_4H_9$ |
| 18 | H | $n-C_3H_7$ |
| 19 | H | $n-C_4H_9$ |
| 20 | F | $C_2H_5$ |
| 21 | H | COOH |

**Patentansprüche**

1. Verbindungen der Formel

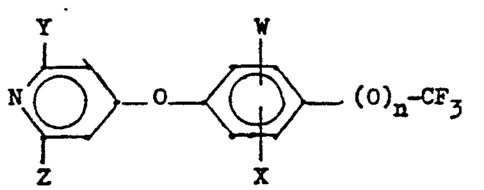

(I)

in der

W und R, die gleich oder verschleden sein können, für H, F, Cl, Br, J, $NO_2$ oder einen gegebenenfalls fluor- oder chlorsubstituierten $C_1$-$C_4$-Alkylrest,

Y für H, F, Cl, Br oder einen gegebenenfalls fluor- oder chlorsubstithierten $C_1$-$C_4$-Alkylrest,

Z für F, Cl, Br, einen gegebenenfalls fluor- oder chlorsubstithierten $C_1$-$C_4$-Alkylrest oder COOH oder

COO- ($C_1$-$C_4$-Alkyl)

und

n für 0 oder 1 steht.

2. Verbindungen nach Anspruch 1, worin W für H, F, Cl, $CH_3$, $CF_3$ oder $NO_2$, X für H, F, Cl, $CH_3$, $CF_3$ oder $NO_2$, Y für H, F, Cl, $CF_3$, $CCl_3$ oder $CH_3$, Z für F, Cl, $CF_3$, $CH_3$, COOH steht.

3. Verbindungen nach Anspruch 1 oder 2, dadurch gekennzeichnet, daß n für die Zahl 0 steht.

4. Biozide, insbesondere herbizide Mittel, gekennzeicknet durch einen Gehalt an einer Verbindung nach Anspruch 1, 2 oder 3.

5. Verwendung von Verbindungen nach Anspruch 1, 2 oder 3 bei der Bekämpfung von Unkräutern.

6. Verfahren zur Herstellung von Verbindungen nach Anspruch 1, dadurch gekennzeichnet, daß man ein Pyridinderivat der Formel

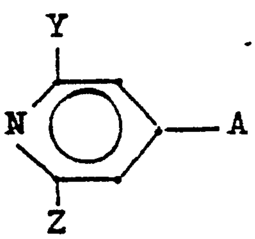

(II),

(A gleich Halogen, insbesondere Chlor oder Fluor, oder $NO_2$)

mit einem Phenol oder Phenolat der Formel

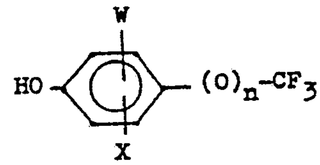

bzw.

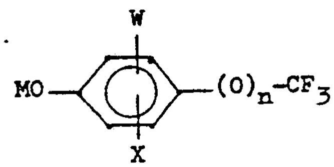

(M gleich 1 Äquivalent eines Kations) umsetzt, wobei im Fall des Phenols ein säurebindendes Mittel zugesetzt wird.

7. Herbizide Mittel, dadurch gekennzeichnet, daß sie als aktive Bestandteile neben einer Verbindung nach Anspruch 1, 2 oder 3 zusätzlich Chlortoluron oder einen verwandten Hannstoff, Terbutryn oder ein verwandtes Triazinderivat, Trifluralin oder eine verwandte Verbindung, Alachlor oder Dimethachlor, 2, 4-DP, MCPA, CMPP, loxynil, Bromoxynil, Bentazon, Brorphenoxim, Dinoterb, Isoproturon oder Dichlofopmethyl enthalten.

8. Verwendung einer Verbindung nach Anspruch 1, 2 oder 3 in Kombination mit Chlortoluron oder einem verwandten Harnstoff, Terbutryn oder einem verwandten Triazinderivat, Trifluralin oder einer verwandten Verbindung, Alachlor oder Dimethachlor, 2, 4-DP, MCPA, CMPP, loxynil, Bromoxynil, Bentazin, Bromphenoxim, Dinoterb, Isoproturon oder Dichlofopmethyl bei der Bekämpfung von Unkräutern.

**Claims**

1. Compounds of formula

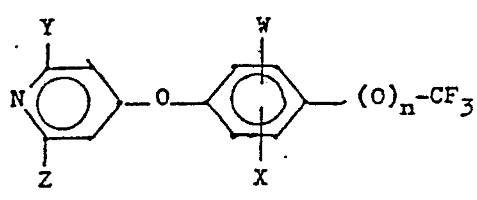

(I)

wherein

W and X, which may be the same or different, represent H, F, Cl, Br, J, $NO_2$ or an optionally fluorineor chlorine-substituted $C_1$-$C_4$ alkyl group,

Y represents H, F, Cl, Br or an optionally fluorineor chlorine-substituted $C_1$-$C_4$ alkyl group,

Z represents F, Cl, Br, an optionally fluorine- or chlorine-substituted $C_1$-$C_4$ alkyl group or COOH

or COO-($C_1$-$C_4$ alkyl)

and

n represents 0 or 1.

2. Compounds as claimed in claim 1 wherein W represents H, F, Cl, $CH_3$, $CF_3$ or $NO_2$, X represents H, F, Cl, $CH_3$, $C_3$ or $NO_2$, Y represents H, F, Cl, $CF_3$, $CCl_3$ or $CH_3$ and Z r;presents F, Cl, $CF_3$, $CH_3$ or COOH.

3. Compounds as claimed in claim 1 or 2, characterised in that n represents the number 0.

4. Biocidal, more particularly herbicidal agents, characterised in that they contain a compound as claimed in claim 1, 2 or 3.

5. Use of compounds as claimed in claim 1, 2 or 3 for controlling weeds.

6. Process for preparing compounds as claimed in claim 1, characterised in that a pyridine derivative of formula

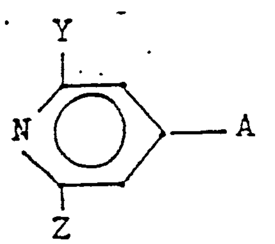

(II)

(wherein A represents halogen particularly chlorine or fluorine, or $NO_2$)

in reacted with a phenol or phenoxide of formula

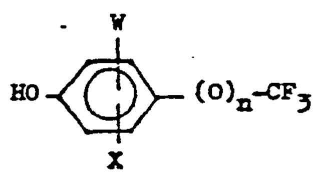

ou

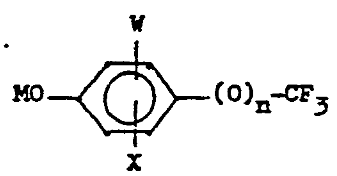

(wherein M equals 1 equivalent of a cation) with an acid-binding agent being added if phenol is used.

7. Herbicidal agents, characterised in that they contain, as active constituents, not only a compound as claimed in claim 1, 2 or 3 but also chlortoluron or a related urea, terbutryn or a related triazine derivative, or trifluralin or a related compound, alachlor or dimethachlor, 2,4-DP, MCPA, CMPP, ioxynil, bromoxynil, bentazone, bromophenoxime, dinoterb, isoproturon or diclofop-methyl.

8. Use of a compound as claimed in claim 1, 2 or 3 in conjunction with chlortoluron or a related urea, terbutryn or a related triazine derivative, trifluralin or a related compound, alachlor or dimethachlor, 2,4-DP, MCPA, CMPP, ioxynil, bromoxynil, bentazine, bromophenoxime, dinoterb, isoproturon or diclofop-methyl for controlling weeds.

**Revendications**

1. Composés de formule

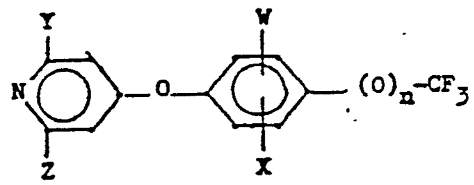

(I)

dans laquelle

W et X qui peuvent être identiques ou différents, remplacent H, F, Cl, Br, I, $NO_2$ ou un radical alcoyle en $C_1$-$C_4$, éventuellement fluoro-ou chlorosubstitué,

Y remplace H, F, Cl, Br ou un radical alcoyle en $C_1$-$C_4$, éventuellement fluoro-ou chlorosubstitué,

Z remplace F, Cl, Br, un radical alcoyle en $C_1$-$C_4$, éventuellement fluoro-ou chlorosubstitué ou COOH ou COO(alcoyle en $C_1$-$C_4$)

et

n remplace 0 ou 1.

2. Composés selon la revendication 1 dans lesquels W remplace H, F, Cl, $CH_3$, $CF_3$ ou $NO_2$, X remplaçe H, F, Cl, $CH_3$, $CF_3$ ou $NO_2$, Y remplace H, F, Cl, $CF_3$, $CCl_3$, ou $CH_3$, Z remplace F, Cl, $CF_3$, $CH_3$, COOH.

3. Composés selon la revendication 1 ou 2, caractérisés en ce que n remplace le chiffre 0.

4. Biocides, en particulier agents herbicides, caractérisés par une teneur en un composé selon la revendication 1, 2 ou 3.

5. Utilisation de composés selon la revendication 1, 2 ou 3 dans la lutte contre les mauvaises herbes.

6. Procédé pour la préparation de composés selon la revendication 1, caractérisé en ce qu'on fait réagir un dérivé de pyridine de formule

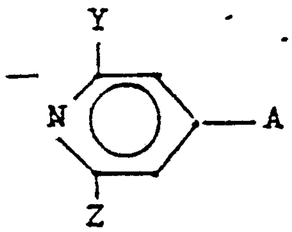 (II),

(A représentant halogène, en particulier chlore ou fluor, ou $NO_2$)
avec un phénol ou phénolate de formule

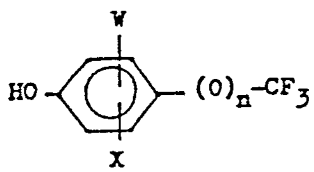 or

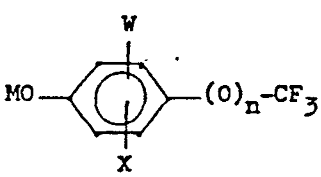

(M représentant un équivalent d'un cation), dans le cas du phénol, un agent fixant les acides étant ajouté.

7. Agents herbicides, caractérisés en ce qu'ils contiennent en tant que constituants actifs, conjointement à un composé selon la revendication 1, 2 ou 3, en outre du chlortoluron ou une urée apparentée, de la terbutryne ou un dérivé de triazine apparenté, de la trifluraline ou un composé apparenté, de l'alachlor ou du diméthachlor, du 2,4-DP, du MCPA, du CMPP, de l'ioxynil, du bromoxynil, de la bentazone, de la bromphénoxime, du dinoterb, de l'isoproturon ou du dichlofopméthyle.

8. Utilisation d'un composé selon la revendication 1, 2 ou 3 en combinaison avec du chlortoluron ou une urée apparentée, de la terbutryne ou un dérivé de triazine apparenté, de la trifluraline ou un composé apparenté, de l'alachlor ou du dimétachlor, du 2,4-DP, du MCPA, du CMPP, de l'ioxynil, du bromoxynil, de la bentazine, de la bromphénoxime, du dinoterb, de l'isoproturon ou du dichlofopméthyle dans la destruction des mauvaises herbes.